# EUROPÄISCHE PATENTSCHRIFT

(11) **EP 3 413 051 B1**
(45) Veröffentlichungstag und Bekanntmachung des Hinweises auf die Patenterteilung: **29.07.2020**
(21) Anmeldenummer: 17175143.1
(22) Anmeldetag: 09.06.2017
(51) Int. Cl.: G01N 33/68, G01N 33/86

(54) **AKTIVIERUNGSTEST ZUR DIAGNOSE EINER HEPARIN-INDUZIERTEN THROMBOZYTOPENIE**
ACTIVATION TEST FOR DIAGNOSING HEPARIN-INDUCED THROMBOCYTOPENIA
TEST D'ACTIVATION DESTINÉ AU DIAGNOSTIC D'UNE THROMBOCYTOPÉNIE INDUITE PAR L'HÉPARINE

(43) Veröffentlichungstag der Anmeldung: 12.12.2018
(73) Patentinhaber: Siemens Healthcare Diagnostics Products GmbH, 35041 Marburg (DE)
(72) Erfinder: Schwarz, Herbert, 35102 LOHRA (DE); Wicke, Michaela, 34560 Fritzlar (DE); Althaus, Harald, 35083 Wetter (DE); Christ, Gerlinde, 35043 Marburg (DE); Zander, Norbert, 35039 Marburg (DE)

(56) Entgegenhaltungen:
- Laila Vengal: "Testing for Heparin-Induced Thrombocytopenia Background Information", , 1. Januar 2016 (2016-01-01), Seiten 1-4, XP055402190, Gefunden im Internet: URL:https://clevelandcliniclabs.com/wp-con tent/uploads/2016/04/anti-platelet-81311.p df [gefunden am 2017-08-29]
- T. E. WARKENTIN ET AL: "The use of well-characterized sera for the assessment of new diagnostic enzyme-immunoassays for the diagnosis of heparin-induced thrombocytopenia", JOURNAL OF THROMBOSIS AND HAEMOSTASIS, Bd. 8, Nr. 1, 1. Januar 2010 (2010-01-01), Seiten 216-218, XP055401734, GB ISSN: 1538-7933, DOI: 10.1111/j.1538-7836.2009.03645.x
- PETRA EICHLER ET AL: "The new ID-heparin/PF4 antibody test for rapid detection of heparin-induced antibodies in comparison with functional and antigenic assays", BRITISH JOURNAL OF HAEMATOLOGY, Bd. 116, Nr. 4, 1. März 2002 (2002-03-01), Seiten 887-891, XP055401729, GB ISSN: 0007-1048, DOI: 10.1046/j.0007-1048.2002.03363.x
- Leo Albrecht ET AL: "Laboratory Diagnosis of Heparin-Induced Thrombocytopenia and Monitoring of Alternative Anticoagulants", CLINICAL AND DIAGNOSTIC LABORATORY IMMUNOLOGY, 1. September 2003 (2003-09-01), Seiten 731-740, XP055015122, DOI: 10.1128/CDLI.10.5.731-740.2003 Gefunden im Internet: URL:http://cvi.asm.org/content/10/5/731.fu ll.pdf#page=1&view=FitH [gefunden am 2011-12-19]

## Beschreibung

Die vorliegende Erfindung liegt auf dem Gebiet der in vitro-Diagnostik und betrifft einen funktionellen, leicht automatisierbaren Test zur Feststellung einer Heparin-induzierten Thrombozytopenie.

Die Heparin-induzierte Thrombozytopenie (HIT) ist eine thrombotische Erkrankung, die im Rahmen einer Heparintherapie entstehen kann und lebensbedrohliche thromboembolische Komplikationen verursachen kann. Betroffene Patienten produzieren Antikörper, die einen Komplex aus Heparin und Plättchenfaktor 4 (PF4) binden, sogenannte anti-PF4/Heparin-Komplex-Antikörper. In vivo bindet der Antikörper-gebundene PF4/Heparin-Komplex an die Thrombozytenoberfläche und verursacht eine Aktivierung der Thrombozyten. Dadurch kommt es zu einer Abnahme der Thrombozytenzahl und einem erhöhten Risiko für Thromboembolien.

Zur HIT-Diagnose werden im Wesentlichen zwei Testprinzipien angewendet. Das erste Testprinzip beruht auf dem direkten Nachweis von anti-PF4/Heparin-Komplex-Antikörpern in einer Körperflüssigkeitsprobe eines Patienten. Dazu wird eine Probe mit PF4/Heparin-Komplex oder einem Komplex aus PF4 und einem anderen geeigneten Polyanion (wie z.B. Polyvinylsulfonat) in Kontakt gebracht, und die Bindung etwaiger in der Probe vorhandener anti-PF4/Heparin-Komplex-Antikörper mit herkömmlichen immunologischen Testverfahren (z.B. ELISA) nachgewiesen (siehe z.B. Warkentin, T.E. et al.: The use of well-characterized sera for the assessment of new diagnostic enzyme-immunoassays for the diagnosis of heparin-induced thrombocytopenia. Journal of Thrombosis and Haemostasis, Vol 8 (1) 2010, 216-218 oder Eichler, P. et al.: The new IDheparin/PF4 antibody test for rapid detection of heparin-induced antibodies in comparison with functional and antigenic assays. British Journal of Haematology, Vol 116 (4) 2002, 887-891). Nachteilig ist jedoch, dass der Nachweis von anti-PF4/Heparin-Komplex-Antikörpern zwar sensitiv, aber nicht ausreichend spezifisch ist, d.h. der Nachweis der Antikörper ist nicht hinreichend für eine positive HIT-Diagnose, während ein negatives Ergebnis eine HIT angemessen sicher ausschließt. Daher wird die Bestätigung durch einen funktionellen Test basierend auf einem zweiten Testprinzip empfohlen (siehe z.B. Vengal, L.: Testing for heparin-induced thrombocytopenia. 2016, URL: https://clevelandcliniclabs.com/ wp-content/uploads/2016/04/anti-platelet-31311.pdf oder Albrecht, L. et al.: Laboratory diagnosis of heparin-induced thrombocytopenia and monitoring of alternative anticoagulants. Clinical and Diagnostic Laboratory Immunology, Vol 10 (5) 2003, 731-740).

Das zweite, funktionelle Testprinzip beruht auf dem Nachweis der Thrombozyten-aktivierenden Wirkung der anti-PF4/Heparin-Komplex-Antikörper. Bei diesem Testprinzip werden gewaschene Thrombozyten von einem oder mehreren normalen Spendern mit einer Plasma- oder Serumprobe eines Patienten und mit Heparin gemischt, und es wird die Thrombozytenaktivierung anhand bekannter Aktivierungsmarker, wie z.B. anhand der Menge von freigesetztem Serotonin (Serotoninfreisetzungstest), oder anhand der visuell erkennbaren Aggregationsreaktion der Thrombozyten (HIPA-Test) gemessen. Enthält eine Patientenprobe anti-PF4/Heparin-Komplex-Antikörper ist eine gegenüber einer normalen Probe (ohne derartige Antikörper) erhöhte Thrombozytenaktivierung feststellbar.

Die beiden genannten funktionellen Testprinzipien können bislang nicht in herkömmlichen Laboren und nicht automatisiert durchgeführt werden, weil sie eine komplexe manuelle Durchführung erfordern und daher nur von geschultem Personal ausgeführt werden können. Darüber hinaus erfordert der Serotoninfreisetzungstest die Abtrennung der Thrombozyten durch einen Zentrifugationsschritt und die Verwendung von radioaktivem Material.

Der vorliegenden Erfindung liegt die Aufgabe zugrunde ein funktionelles Verfahren zur Detektion von anti-PF4/Heparin-Komplex-Antikörpern in einer Körperflüssigkeitsprobe bereit zu stellen, bei dem die vorgenannten Nachteile vermieden werden.

Es wurde gefunden, dass durch die quantitative Bestimmung von PF4 in einem Reaktionsgemisch, welches die zu untersuchende Probe, Thrombozyten und Heparin (oder ein funktionell gleichwertiges, PF4-bindendes Polysaccharid oder Polyanion) enthält, festgestellt werden kann, ob die Probe anti-PF4/Heparin-Komplex-Antikörper enthält. Proben, die anti-PF4/Heparin-Komplex-Antikörper enthalten, verursachen in dem genannten Reaktionsgemisch offenbar eine PF4-Ausschüttung in den Thrombozyten, so dass eine erhöhte PF4-Menge in dem Reaktionsgemisch das Vorhandensein von anti-PF4/Heparin-Komplex-Antikörpern anzeigt und damit das Vorliegen einer Heparin-induzierten Thrombozytopenie.

Gegenstand der vorliegenden Erfindung ist also ein Verfahren zur Detektion von anti-PF4/Heparin-Komplex-Antikörpern in einer Körperflüssigkeitsprobe. Das Verfahren umfasst die Schritte:
i. Bereitstellen eines Reaktionsgemisches durch Vermischen der Probe mit einem Thrombozyten-haltigen Reagenz und mit einem PF4-bindenden, unverzweigten Polysaccharid oder mit einem PF4-bindenden Polyanion;
ii. Inkubation des Reaktionsgemisches; und dann
iii. Bestimmen der Menge von PF4 in dem Reaktionsgemisch;
iv. Vergleichen der so bestimmten Menge von PF4 in dem Reaktionsgemisch mit einem vorbestimmten Referenzwert für die Menge von PF4 in Reaktionsgemischen enthaltend Körperflüssigkeitsproben von Spendern, die bekanntermaßen keine anti-PF4/Heparin-Komplex-Antikörper enthalten; und
v. Feststellen des Vorhandenseins von anti-PF4/Heparin-Komplex-Antikörpern in der Probe, wenn die in dem Reaktionsgemisch bestimmte Menge von PF4 den Referenzwert überschreitet.

Die Körperflüssigkeitsprobe stammt bevorzugterweise von einem Menschen. Bevorzugterweise handelt es sich um eine im Wesentlichen Thrombozyten-freie Körperflüssigkeitsprobe, insbesondere um Plasma oder Serum.

Das für die Bereitstellung des Reaktionsgemisches benötigte Thrombozyten-haltige Reagenz ist vorzugsweise eine Suspension gewaschener und resuspendierter humaner Thrombozyten. Die Thrombozyten stammen vorzugsweise von einem oder mehreren gesunden Spendern, die bekanntermaßen keine anti-PF4/Heparin-Komplex-Antikörper enthalten. Zur Herstellung eines geeigneten Thrombozyten-haltigen Reagenzes werden beispielsweise Citrat-Vollblutproben, denen vorzugsweise auch Hirudin zugegeben wurde, zentrifugiert, um plättchenreiches Plasma zu gewinnen. Dem plättchenreichen Plasma wird nochmals Citratlösung und Apyrase zugesetzt, es wird nochmals zentrifugiert, und anschließend wird der zellfreie Überstand verworfen. Die pelletierten Thrombozyten werden schließlich in einer gepufferten Suspensionslösung aufgenommen.

Die Körperflüssigkeitsprobe wird ferner mit einem PF4-bindenden, unverzweigten Polysaccharid oder mit einem PF4-bindenden Polyanion vermischt. Es sind eine Vielzahl PF4-bindender Substanzen bekannt, die mit PF4 einen Komplex ausbilden, der von den zu detektierenden anti-PF4/Heparin-Komplex-Antikörpern gebunden wird. Geeignete unverzweigte Polysaccharide sind beispielsweise Heparin, unfraktioniertes Heparin (UFH), fraktioniertes Heparin (LMWH), Dextransulfat und Fucoidan. Geeignete Polyanionen sind beispielsweise Polyvinylsulfat, Polyvinylsulfonat, Polyvinylphosphat, Polyvinylphosphonat, Polystyrensulfat und Polystyrensulfonat.

Vor der Bestimmung der PF4-Menge in dem Reaktionsgemisch wird das Reaktionsgemisch für einen Zeitraum inkubiert, um die Komplexbildung zwischen dem PF4-bindenden, unverzweigten Polysaccharid oder Polyanion und dem in der Probe vorhandenen PF4-Protein, die Bindung der vermutlich in der Probe enthaltenen anti-PF4/Heparin-Komplex-Antikörper an den entstandenen Komplex und schließlich die Aktivierung der Thrombozyten durch die Bindung des Antikörper-gebundenen Komplexes an der Thrombozytenoberfläche zu ermöglichen. Eine typische Inkubationsdauer beträgt zwischen 5 und 10 Minuten, vorzugsweise bei +37 °C.

Nach ausreichender Inkubation werden Schritte durchgeführt, um die Menge von PF4 in dem Reaktionsgemisch zu bestimmen. Die Bestimmung der Menge von PF4 in dem Reaktionsgemisch kann auf unterschiedliche Art und Weise erfolgen. Bevorzugt sind Bindungsteste, bei denen zum Bestimmen der Menge von PF4 in dem Reaktionsgemisch mindestens ein PF4-Bindungspartner, wie z.B. ein anti-PF4-Antikörper oder Heparin oder ein PF4-bindendes, unverzweigtes Polysaccharid oder ein PF4-bindendes Polyanion, mit dem Reaktionsgemisch in Kontakt gebracht wird. Dies kann beispielsweise dadurch erfolgen, dass das Reaktionsgemisch (ganz oder teilweise) mit einem an eine Festphase assoziierten PF4-Bindungspartner in Kontakt gebracht wird. Ungebundene Bestandteile werden durch Waschen entfernt, und mit Hilfe eines zweiten, markierten PF4-Bindungspartners wird die Menge des an die Festphase gebundenen PF4-Proteins bestimmt (ELISA-Technik). Besonders geeignete anti-PF4-Antikörper sind monoklonale oder polyklonale Antikörper mit Spezifität für freies PF4, d.h. mit Spezifität für PF4, das nicht an Heparin oder ein funktionell gleichwertiges Polysaccharid oder Polyanion gebunden ist, oder monoklonale oder polyklonale Antikörper, die sowohl freies als auch komplexiertes PF4-Protein binden.

Besonders bevorzugt sind homogene Immunteste, mit denen auf die Entfernung ungebundener Bestandteile und damit auf Waschschritte verzichtet werden kann. Dazu wird das Reaktionsgemisch (ganz oder teilweise) mit einem ersten und einem zweiten anti-PF4-Antikörper und mit einer ersten und einer zweiten Komponente eines signalbildenden Systems vermischt. Die Komponenten des signalbildenden Systems wirken derart zusammen, dass ein detektierbares Signal entsteht, wenn die erste und die zweite Komponente des signalbildenden Systems in räumliche Nähe zueinander gebracht werden. Dabei ist der erste anti-PF4-Antikörper mit der ersten Komponente des signalbildenden Systems assoziiert oder wird während der Inkubation des Reaktionsgemisches damit assoziiert, und der zweite anti-PF4-Antikörper ist mit der zweiten Komponente des signalbildenden Systems assoziiert oder wird während der Inkubation des Reaktionsgemisches damit assoziiert.

Bei einer Ausführungsform eines homogenen PF4-Immuntestes handelt es sich bei den Komponenten des signalbildenden Systems um partikuläre Festphasen, beispielsweise Latexpartikel, deren Agglutination turbidimetrisch oder nephelometrisch bestimmt wird. Dazu besteht die erste Komponente des signalbildenden Systems aus einer ersten partikulären Festphase, die so beschaffen ist, dass sie mit dem ersten anti-PF4-Antikörper assoziiert ist oder assoziierbar ist. Die erste partikuläre Festphase kann mit dem ersten anti-PF4-Antikörper über eine kovalente Bindung oder über ein Bindungspaar X/Y verbunden sein oder über ein Bindungspaar X/Y im Reaktionsansatz verbunden werden. Weiterhin besteht die zweite Komponente des signalbildenden Systems aus einer zweiten partikulären Festphase, die so beschaffen ist, dass sie mit dem zweiten anti-PF4-Antikörper assoziiert ist oder assoziierbar ist. Die zweite partikuläre Festphase kann mit dem zweiten anti-PF4-Antikörper über eine kovalente Bindung oder über ein Bindungspaar A/B verbunden sein oder über ein Bindungspaar A/B im Reaktionsansatz verbunden werden. Immunoassays beruhend auf dem Prinzip der partikelverstärkten Lichtstreuung sind seit etwa 1920 bekannt (zur Übersicht siehe Newman, D.J. et al., Particle enhanced light scattering immunoassay. Ann Clin Biochem 1992; 29: 22-42). Bevorzugterweise werden Polystyrolpartikel mit einem Durchmesser von 0,1 bis 0,5 µm, besonders bevorzugt mit einem Durchmesser von 0,15 bis 0,35 µm verwendet. Bevorzugt werden Polystyrolpartikel mit Amin-, Carboxyl- oder Aldehydfunktionen verwendet. Weiterhin bevorzugt werden Schale/Kern-Partikel verwendet. Die Synthese der Partikel und die kovalente Kopplung von Liganden ist z.B. in Peula, J.M. et al., Covalent coupling of antibodies to aldehyde groups on polymer carriers. Journal of Materials Science: Materials in Medicine 1995; 6: 779-785 beschrieben.

In einer anderen Ausführungsform eines homogenen PF4-Immuntestes umfasst das signalbildende System mindestens eine erste und eine zweite Komponente, welche so zusammenwirken, dass ein detektierbares Signal entsteht, wenn sie in räumliche Nähe zueinander gebracht werden und dadurch miteinander in Wechselwirkung treten können. Unter einer Wechselwirkung zwischen den Komponenten ist insbesondere ein Energietransfer - also die direkte Übertragung von Energie zwischen den Komponenten, z.B. durch Licht- oder Elektronenstrahlung sowie über reaktive chemische Moleküle, wie z. B. kurzlebigen Singulett-Sauerstoff - zu verstehen. Der Energietransfer kann von einer auf eine andere Komponente erfolgen, möglich ist aber auch eine Kaskade verschiedener Substanzen über die der Energietransfer läuft. Zum Beispiel kann es sich bei den Komponenten um ein Paar aus einem Energiespender und einem Energieempfänger handeln, wie beispielsweise Photosensitizer und chemilumineszierendes Agens (EP-A2-0515194, LOCI® Technologie) oder Photosensitizer und Fluorophor (WO 95/06877) oder radioaktives Iod<125> und Fluorophor (Udenfriend et al. (1985) Proc. Natl. Acad. Sci. 82: 8672 - 8676) oder Fluorophor und Fluoreszenz-Quencher (US 3,996,345). Besonders bevorzugt ist die erste Komponente des signalbildenden Systems ein chemilumineszierendes Agens und die zweite Komponente des signalbildenden Systems ein Photosensitizer oder umgekehrt, und es wird die Chemilumineszenz im Reaktionsgemisch gemessen.

Die erste Komponente und/oder die zweite Komponente des signalbildenden Systems, die miteinander in Wechselwirkung treten können, können mit einer partikulären Festphase kovalent oder über spezifische Wechselwirkung assoziiert oder in diese eingelagert sein. Unter dem Begriff partikuläre Festphase sind nicht-zelluläre, suspendierbare Teilchen zu verstehen, wie z.B. Metallsolen, Silica-Partikel, Magnetpartikel oder besonders bevorzugt Latexpartikel. Bevorzugt werden Partikel mit einem Durchmesser von 0,01 - 10 µm, insbesondere bevorzugt werden Partikel mit einem Durchmesser von 0,1 - 1 µm.

Die erste Komponente des signalbildenden Systems, dessen Komponenten so zusammenwirken, dass ein detektierbares Signal entsteht, wenn sie in räumliche Nähe zueinander gebracht werden und dadurch miteinander in Wechselwirkung treten können, ist so beschaffen, dass sie mit dem ersten anti-PF4-Antikörper assoziiert ist oder assoziierbar ist. Die erste Komponente des signalbildenden Systems kann direkt mit dem ersten anti-PF4-Antikörper assoziiert sein oder assoziierbar sein. Bevorzugterweise ist die erste Komponente des signalbildenden Systems indirekt mit dem ersten anti-PF4-Antikörper assoziiert oder assoziierbar. Dazu ist die erste Komponente des signalbildenden Systems mit einer partikulären Festphase assoziiert, welche zusätzlich mit dem ersten anti-PF4-Antikörper kovalent oder über ein Bindungspaar X/Y assoziiert ist oder über ein Bindungspaar X/Y assoziierbar ist.

Die zweite Komponente des signalbildenden Systems, dessen Komponenten so zusammenwirken, dass ein detektierbares Signal entsteht, wenn sie in räumliche Nähe zueinander gebracht werden und dadurch miteinander in Wechselwirkung treten können, ist so beschaffen, dass sie mit dem zweiten anti-PF4-Antikörper assoziiert ist oder assoziierbar ist. Die zweite Komponente des signalbildenden Systems kann direkt mit dem zweiten anti-PF4-Antikörper assoziiert sein oder assoziierbar sein. Bevorzugterweise ist die zweite Komponente des signalbildenden Systems indirekt mit dem zweiten anti-PF4-Antikörper assoziiert oder assoziierbar. Dazu ist die zweite Komponente des signalbildenden Systems mit einer partikulären Festphase assoziiert, welche zusätzlich mit dem Liganden kovalent oder über ein Bindungspaar A/B assoziiert ist oder über ein Bindungspaar A/B assoziierbar ist.

Die "Bindungspartner X und Y" beziehungsweise die "Bindungspartner A und B" sind jeweils zwei unterschiedliche Moleküle, die einander spezifisch erkennen und binden. Beispiele für spezifische Erkennung und Bindung sind Antikörper-Antigen-Wechselwirkungen, Polynukleotid-Wechselwirkungen etc.

Geeignete Bindungspaare X/Y beziehungsweise A/B sind vor allem Antigen/Antikörper-Kombinationen, wobei der Bindungspartner X oder A ein antigenes Epitop des anti-PF4-Antikörpers ist. Das antigene Epitop kann ein natürliches Sequenz- oder Strukturepitop des Antikörpers sein. Das antigene Epitop kann auch ein heterologes Sequenz- oder Strukturepitop eines modifizierten anti-PF4-Antikörpers sein. Beispiele für heterologe Sequenz- oder Strukturepitope sind FLAG- oder HIS- oder Fluorescein-Tags, die insbesondere zur Markierung von Peptiden oder Proteinen verwendet werden. Weitere geeignete Bindungspaare X/Y beziehungsweise A/B sind komplementäre Polynukleotide X und Y beziehungsweise A und B. Besonders bevorzugte Bindungspaare X/Y beziehungsweise A/B sind FLAG-Tag/anti-FLAG-Tag-Antikörper, HIS-Tag/anti-HIS-Tag-Antikörper Fluorescein/anti-Fluorescein-Antikörper, Biotin/Avidin und Biotin/Streptavidin.

Es wurde gefunden, dass mit den beschriebenen homogenen Immuntesten auf einen Zentrifugationsschritt zur Abtrennung der Thrombozyten in dem Reaktionsgemisch verzichtet werden kann. Dies stellt eine Vereinfachung des Verfahrensablaufs dar, wodurch eine automatische Abarbeitung des Verfahrens in gängigen Analysegeräten ermöglicht wird. In einer bevorzugten Ausführungsform des erfindungsgemäßen Verfahrens wird das bereitgestellte Reaktionsgemisch keinem Zentrifugationsschritt zur Sedimentation oder Abtrennung der Thrombozyten unterworfen.

Nachdem die PF4-Menge in dem Reaktionsgemisch bestimmt wurde, wird die so bestimmte Menge von PF4 mit einem vorbestimmten Referenzwert verglichen. Als Referenzwert eignet sich die Menge von PF4, die mit demselben Verfahren in Reaktionsgemischen bestimmt wird (oder vorab bestimmt wurde), die Körperflüssigkeitsproben von Spendern enthalten, die bekanntermaßen keine anti-PF4/Heparin-Komplex-Antikörper enthalten. Üblicherweise wird zur Bestimmung eines Referenzwertes in einer Vielzahl von Proben von gesunden Spendern, die bekanntermaßen keine anti-PF4/Heparin-Komplex-Antikörper aufweisen, die PF4-Menge bestimmt und dann mit der PF4-Menge einer Vielzahl von Proben von an HIT erkrankten Spendern, die anti-PF4/Heparin-Komplex-Antikörper aufweisen, verglichen. Ein Referenzwert kann beispielsweise dann ein Grenzwert sein, der die Differenzierung von Proben mit und Proben ohne anti-PF4/Heparin-Komplex-Antikörpern ermöglicht. Überschreitet die in einem Reaktionsgemisch bestimmte Menge von PF4 den Referenzwert, erlaubt dies das Feststellen des Vorhandenseins von anti-PF4/Heparin-Komplex-Antikörpern in der Probe. Wenn die in dem Reaktionsgemisch bestimmte Menge von PF4 den Referenzwert hingegen unterschreitet, erlaubt dies das Feststellen des Fehlens von anti-PF4/Heparin-Komplex-Antikörpern in der Probe.

Ein weiterer Gegenstand der vorliegenden Erfindung ist ein Verfahren zur Diagnose einer Heparin-induzierten Thrombozytopenie, wobei mit einem erfindungsgemäßen Verfahren das Vorhandensein von anti-PF4/Heparin-Komplex-Antikörpern in einer Körperflüssigkeitsprobe eines Patienten detektiert wird.

Noch ein weiterer Gegenstand der vorliegenden Erfindung ist ein Testkit zur Durchführung eines erfindungsgemäßen Verfahrens. Das Testkit enhält mindestens folgende Komponenten:
a. ein erstes Reagenz enthaltend Thrombozyten;
b. ein zweites Reagenz enthaltend ein PF4-bindendes, unverzweigtes Polysaccharid oder ein PF4-bindendes Polyanion; und
c. ein oder mehrere Reagenzien zum Nachweis von PF4, wobei mindestens ein Reagenz einen anti-PF4-Antikörper enthält.

Das Reagenz enthaltend Thrombozyten ist vorzugsweise eine Suspension gewaschener und resuspendierter humaner Thrombozyten. Die Thrombozyten stammen vorzugsweise von einem oder mehreren gesunden Spendern, die bekanntermaßen keine anti-PF4/Heparin-Komplex-Antikörper enthalten. Zur Herstellung eines geeigneten Thrombozyten-haltigen Reagenzes werden beispielsweise Citrat-Vollblutproben, denen vorzugsweise auch Hirudin zugegeben wurde, zentrifugiert, um plättchenreiches Plasma zu gewinnen. Dem plättchenreichen Plasma wird nochmals Citratlösung und Apyrase zugesetzt, es wird nochmals zentrifugiert, und anschließend wird der zellfreie Überstand verworfen. Die pelletierten Thrombozyten werden schließlich in einer gepufferten Suspensionslösung aufgenommen. Ein geeignetes Thrombozyten-haltiges Reagenz enthält mindestens 300 x 10⁹ Thrombozyten pro Liter.

Das zweite Reagenz enthält entweder
- ein PF4-bindendes, unverzweigtes Polysaccharid, bevorzugt aus der Gruppe Heparin, unfraktioniertes Heparin, fraktioniertes Heparin, Dextransulfat und Fucoidan; oder
- ein PF4-bindendes Polyanion, bevorzugt aus der Gruppe Polyvinylsulfat, Polyvinylsulfonat, Polyvinylphosphat, Polyvinylphosphonat, Polystyrensulfat und Polystyrensulfonat.

Das erste und das zweite Reagenz sind zur Bereitstellung des Reaktionsgemisches mit der Körperflüssigkeitsprobe vorgesehen.

Das Testkit enthält außerdem ein oder mehrere Reagenzien zum Nachweis von PF4, wobei mindestens ein Reagenz einen anti-PF4-Antikörper enthält. Bei einem Reagenz, das einen anti-PF4-Antikörper enthält, kann es sich um einen Festphasenassoziierten Antikörper handeln, beispielsweise um einen an Latexpartikel oder in der Vertiefung einer Mikrotitrationsplatte gebundenen Antikörper. Je nach Testformat enthält ein Testkit zusätzliche Komponenten zum quantitativen Nachweis von anti-PF4-Antikörper-gebundenem PF4, wie z.B. ein Reagenz enthaltend einen mit einem nachweisbaren Label markierten Zweitantikörper.

Ein bevorzugtes Testkit enthält ein Reagenz enthaltend einen ersten anti-PF4-Antikörper und ein anderes Reagenz enthaltend einen zweiten anti-PF4-Antikörper. Der erste und/oder der zweite anti-PF4-Antikörper können mit einer Festphase und/oder einer ersten beziehungsweise zweiten Komponente eines signalbildenden Systems, dessen Komponenten so zusammenwirken, dass ein detektierbares Signal entsteht, wenn sie in räumliche Nähe zueinander gebracht werden, assoziiert sein.

Die Reagenzien eines erfindungsgemäßen Testkits können in flüssiger oder lyophilisierter Form bereitgestellt werden. Für den Fall, dass einige oder alle Reagenzien des Testkits als Lyophilisate vorliegen, kann das Testkit zusätzlich die zur Lösung der Lyophilisate erforderlichen Lösemittel enthalten, wie z.B. destilliertes Wasser oder geeignete Puffer.

Die folgenden Beispiele dienen der Veranschaulichung der vorliegenden Erfindung und sind nicht als Einschränkung zu verstehen.

### BEISPIELE

### Beispiel 1: Homogener Immunoassay zur Detektion von anti-PF4/Heparin-Komplex-Antikörpern

Die hier verwendete LOCI®-Technologie beruht darauf, dass eine Latexpartikel-gekoppelte Chemilumineszenzverbindung (Chemibeads) und ein Latexpartikel-gekoppelter Photosensibilisator (Sensibeads) durch gleichzeitige Bindung an einen Analyten in eine räumliche Nähe zueinander gebracht werden, so dass Singulett-Sauerstoff, der vom Photosensibilisator erzeugt wird, die Chemilumineszenzverbindung anregen kann.

2 µL einer humanen, Hitze-inaktivierten (bei 56 °C, 30 min) Plasmaprobe wurden mit 7,5 µL einer Suspension enthaltend humane, gewaschene Thrombozyten von 6 gesunden Spendern (ca. 300 x 10⁹ Thrombozyten/L) und 10 µL einer Pufferlösung enthaltend 0,2 IU/mL Heparin gemischt und bei 37 °C inkubiert. Nach 10 Minuten wurden dem Reaktionsgemisch folgende Komponenten zugegeben:
- 50 µL einer "Chemibead"-Lösung enthaltend in einer Pufferlösung Latexpartikel, die mit einer Chemilumineszenzverbindung (2-(4-(N,N, di- tetradecyl)-anilino-3-phenyl thioxen) und einem ersten monoklonalen anti-PF4-Antikörper (MAK-23/064), der sowohl freies als auch komplexiertes PF4-Protein bindet, beschichtet sind (50 µg/mL);
- 50 µL einer Antikörperlösung enthaltend einen zweiten biotinylierten monoklonalen anti-PF4-Antikörper (MAK-23/074)), der sowohl freies als auch komplexiertes PF4-Protein bindet (5 µg/mL); und
- 100 µL einer "Sensibead"-Lösung enthaltend in einer Pufferlösung Latexpartikel, die mit einer Photosensibilisatorverbindung (bis-(trihexyl)-silicon-t-butyl-phthalocyanine) und Streptavidin beschichtet sind (50 µg/mL).

Nach etwa 10 Minuten wurde das Chemilumineszenzsignal gemessen [kcounts].

Mit dem erfindungsgemäßen Verfahren (im Folgenden auch "PF4-Release" genannt) wurden Plasmaproben von 10 HIT-Patienten gemessen, bei denen anhand klinischer Kriterien (4T-Score, zum Teil mit thrombotischem Ereignis) eine HIT diagnostiziert und das Vorliegen von anti-PF4/Heparin-Komplex-Antikörpern mit zwei unabhängigen, kommerziell erhältlichen Immunoassays (HemosIL® AcuStar HIT-Ab(PF4-H), Instrumentation Laboratories und Asserachrom® HPIA-IgG, Diagnostica Stago) festgestellt worden war.

Mit dem erfindungsgemäßen Verfahren wurden ferner Plasmaproben von 7 gesunden Spendern (die keine klinischen HIT-Kriterien und auch keine anti-PF4/Heparin-Komplex-Antikörper aufwiesen) und ein normaler Plasmapool (aus etwa 20 Plasmen gesunder Spender, "FNP") gemessen.

Als "100 %-Kontrolle" für den PF4-Release-Test wurde anstelle einer Plasmaprobe eine Antikörperlösung enthaltend einen Thrombozyten-aktivierenden anti-PF4/Heparin-Komplex-Antikörper (50 µg/mL) eingesetzt, um die maximal mögliche PF4-Ausschüttung mit dem verwendeten Thrombozyten-haltigen Reagenz zu ermitteln. Zur Ermittlung der PF4-Ausschüttung in % (PF4-Release [%]) wurden die in kcounts gemessenen Rohwerte zu dem Rohwert der 100 %-Kontrolle ins Verhältnis gesetzt.

Die genannten Proben wurden zu Vergleichszwecken außerdem mit dem [14C]-Serotonin-Release Assay (im Folgenden auch "SRA" genannt) gemessen, nach Sheridan, D. et al. (1986) A diagnostic test for heparin-induced thrombocytopenia. Blood 67(1): 27-30, der als Goldstandard gilt.

In Tabelle 1 sind die Testergebnisse zusammengefasst.

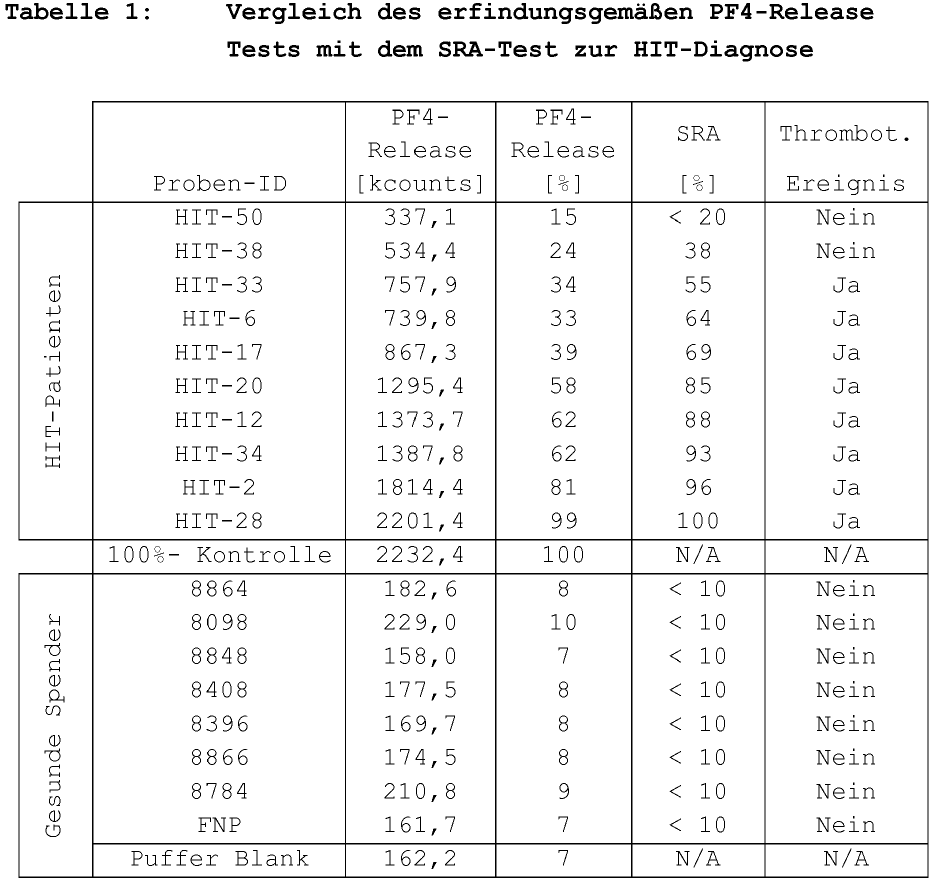

Es zeigt sich, dass die Ergebnisse des erfindungsgemäßen Verfahrens sehr gut mit den Ergebnissen des SRA-Goldstandard-Tests korrelieren. In allen Reaktionsgemischen enthaltend HIT-Patientenproben ist eine gegenüber gesunden Spendern signifikant erhöhte PF4-Konzentration nachweisbar. Als Grenzwert (Cut-Off) für die Differenzierung von Proben, die anti-PF4/Heparin-Komplex-Antikörper enthalten, von solchen die keine enthalten, könnte ein PF4-Release-Wert von ≥ 15 % oder auch ≥ 20 % festgelegt werden (Cut-Off SRA-Test: ≥ 20%). Für eine statistisch genauere Festlegung des Cut-Off-Wertes ist jedoch eine deutliche höhere Anzahl von Probenmessungen erforderlich.

## Patentansprüche

1. Verfahren zur Detektion von anti-PF4/Heparin-Komplex-Antikörpern in einer Körperflüssigkeitsprobe, das Verfahren umfassend die Schritte:
i. Bereitstellen eines Reaktionsgemisches durch Vermischen der Probe mit einem Thrombozyten-haltigen Reagenz und mit einem PF4-bindenden, unverzweigten Polysaccharid oder mit einem PF4-bindenden Polyanion;
ii. Inkubation des Reaktionsgemisches; und dann
iii. Bestimmen der Menge von PF4 in dem Reaktionsgemisch;
iv. Vergleichen der so bestimmten Menge von PF4 in dem Reaktionsgemisch mit einem vorbestimmten Referenzwert für die Menge von PF4 in Reaktionsgemischen enthaltend Körperflüssigkeitsproben von Spendern, die bekanntermaßen keine anti-PF4/Heparin-Komplex-Antikörper enthalten; und
v. Feststellen des Vorhandenseins von anti-PF4/Heparin-Komplex-Antikörpern in der Probe, wenn die in dem Reaktionsgemisch bestimmte Menge von PF4 den Referenzwert überschreitet.

2. Verfahren gemäß Anspruch 1, wobei das Thrombozyten-haltige Reagenz humane Thrombozyten von einem oder mehreren gesunden Spendern, die bekanntermaßen keine anti-PF4/Heparin-Komplex-Antikörper enthalten, enthält.

3. Verfahren gemäß einem der Ansprüche 1 und 2, wobei zum Bestimmen der Menge von PF4 in dem Reaktionsgemisch mindestens ein anti-PF4-Antikörper mit dem Reaktionsgemisch in Kontakt gebracht wird.

4. Verfahren gemäß Anspruch 3, wobei
• ein erster und ein zweiter anti-PF4-Antikörper und
• eine erste und eine zweite Komponente eines signalbildenden Systems, welche so zusammenwirken, dass ein detektierbares Signal entsteht, wenn die erste und die zweite Komponente des signalbildenden Systems in räumliche Nähe zueinander gebracht werden,
mit dem Reaktionsgemisch vermischt werden und wobei der erste anti-PF4-Antikörper mit der ersten Komponente des signalbildenden Systems assoziiert ist oder während der Inkubation des Reaktionsgemisches assoziiert wird und wobei der zweite anti-PF4-Antikörper mit der zweiten Komponente des signalbildenden Systems assoziiert ist oder während der Inkubation des Reaktionsgemisches assoziiert wird.

5. Verfahren gemäß Anspruch 4, wobei die erste und zweite Komponente des signalbildenden Systems jeweils eine partikuläre Festphase umfassen, und wobei die Agglutination der partikulären Festphasen im Reaktionsgemisch gemessen wird.

6. Verfahren gemäß Anspruch 4, wobei die erste Komponente des signalbildenden System ein chemilumineszierendes Agens ist und die zweite Komponente des signalbildenden Systems ein Photosensitizer ist oder umgekehrt und wobei die Chemilumineszenz im Reaktionsgemisch gemessen wird.

7. Verfahren gemäß einem der Ansprüche 4 bis 6, bei dem das bereitgestellte Reaktionsgemisch keinem Zentrifugationsschritt zur Abtrennung der Thrombozyten unterworfen wird.

8. Verfahren zur Diagnose einer Heparin-induzierten Thrombozytopenie, wobei mit einem Verfahren gemäß einem der Ansprüche 1 bis 7 das Vorhandensein von anti-PF4/Heparin-Komplex-Antikörpern in einer Körperflüssigkeitsprobe eines Patienten detektiert wird.

9. Testkit zur Durchführung eines Verfahrens gemäß einem der Ansprüche 1 bis 8, welches folgende Komponenten enthält:
a. ein erstes Reagenz enthaltend Thrombozyten;
b. ein zweites Reagenz enthaltend ein PF4-bindendes, unverzweigtes Polysaccharid oder ein PF4-bindendes Polyanion; und
c. ein oder mehrere Reagenzien zum Nachweis von PF4, wobei mindestens ein Reagenz einen anti-PF4-Antikörper enthält.

10. Testkit gemäß Anspruch 9, wobei das zweite Reagenz
• ein PF4-bindendes, unverzweigtes Polysaccharid aus der Gruppe Heparin, unfraktioniertes Heparin, fraktioniertes Heparin, Dextransulfat und Fucoidan enthält; oder
• ein PF4-bindendes Polyanion aus der Gruppe Polyvinylsulfat, Polyvinylsulfonat, Polyvinylphosphat, Polyvinylphosphonat, Polystyrensulfat und Polystyrensulfonat enthält.

## Claims

1. Method for detecting anti-PF4/heparin complex antibodies in a body-fluid sample, the method comprising the steps:
i. providing a reaction mixture by mixing the sample with a thrombocyte-containing reagent and with a PF4-binding, unbranched polysaccharide or with a PF4-binding polyanion;
ii. incubating the reaction mixture; and then
iii. determining the amount of PF4 in the reaction mixture;
iv. comparing the thus determined amount of PF4 in the reaction mixture with a predetermined reference value for the amount of PF4 in reaction mixtures containing body-fluid samples from donors known to contain no anti-PF4/heparin complex antibodies; and
v. establishing the presence of anti-PF4/heparin complex antibodies in the sample when the amount of PF4 that is determined in the reaction mixture exceeds the reference value.

2. Method according to Claim 1, wherein the thrombocyte-containing reagent contains human thrombocytes from one or more healthy donors known to contain no anti-PF4/heparin complex antibodies.

3. Method according to either of Claims 1 and 2, wherein the amount of PF4 in the reaction mixture is determined by contacting at least one anti-PF4 antibody with the reaction mixture.

4. Method according to Claim 3, wherein
• a first and a second anti-PF4 antibody and
• a first and a second component of a signal-forming system, which interact such that a detectable signal is formed when the first and the second component of the signal-forming system are brought into close proximity to one another,
are mixed with the reaction mixture and wherein the first anti-PF4 antibody is in an associated state with the first component of the signal-forming system or is associated therewith during the incubation of the reaction mixture and wherein the second anti-PF4 antibody is in an associated state with the second component of the signal-forming system or is associated therewith during the incubation of the reaction mixture.

5. Method according to Claim 4, wherein the first and second component of the signal-forming system comprise in each case a particulate solid phase, and wherein the agglutination of the particulate solid phases in the reaction mixture is measured.

6. Method according to Claim 4, wherein the first component of the signal-forming system is a chemiluminescent agent and the second component of the signal-forming system is a photosensitizer or vice versa and wherein the chemiluminescence in the reaction mixture is measured.

7. Method according to any of Claims 4 to 6, in which the provided reaction mixture is not subjected to a centrifugation step for the removal of the thrombocytes.

8. Method for diagnosing a heparin-induced thrombocytopenia, wherein a method according to any of Claims 1 to 7 is used to detect the presence of anti-PF4/heparin complex antibodies in a body-fluid sample from a patient.

9. Assay kit for carrying out a method according to any of Claims 1 to 8, containing the following components:
a. a first reagent containing thrombocytes;
b. a second reagent containing a PF4-binding, unbranched polysaccharide or a PF4-binding polyanion; and
c. one or more reagents for the detection of PF4, with at least one reagent containing an anti-PF4 antibody.

10. Assay kit according to Claim 9, wherein the second reagent
• contains a PF4-binding, unbranched polysaccharide from the group consisting of heparin, unfractionated heparin, fractionated heparin, dextran sulfate and fucoidan; or
• contains a PF4-binding polyanion from the group consisting of polyvinyl sulfate, polyvinyl sulfonate, polyvinyl phosphate, polyvinyl phosphonate, polystyrene sulfate and polystyrene sulfonate.

## Revendications

1. Procédé de détection d'anticorps de complexe anti-PF4/héparine dans un échantillon de liquide corporel, le procédé comprenant les stades :
i. préparation d'un mélange réactionnel en mélangeant l'échantillon à un réactif contenant des thrombocytes et à un polysaccharide non ramifié fixant PF4 ou à un polyanion fixant PF4 ;
ii. incubation du mélange réactionnel ; et ensuite
iii. détermination de la quantité de PF4 dans le mélange réactionnel ;
iv. comparaison de la quantité de PF4 ainsi déterminée dans le mélange réactionnel à une valeur de référence déterminée à l'avance de la quantité de PF4 dans le mélange réactionnel contenant des échantillons de liquide corporel de donneurs, qui, de manière connue, ne contiennent pas d'anticorps de complexe anti-PF4/héparine ; et
v constatation de la présence d'anticorps de complexe anti-PF4/héparine dans l'échantillon, si la quantité de PF4 déterminée dans le mélange réactionnel dépasse la valeur de référence.

2. Procédé suivant la revendication 1, dans lequel le réactif contenant des thrombocytes contient des thrombocytes humains d'un donneur ou de plusieurs donneurs sains, qui, d'une manière connue, ne contiennent pas d'anticorps de complexe anti-PF4/héparine.

3. Procédé suivant l'une des revendications 1 et 2, dans lequel, pour déterminer la quantité de PF4 dans le mélange réactionnel, on met au moins un anticorps anti-PF4 en contact avec le mélange réactionnel.

4. Procédé suivant la revendication 3, dans lequel on mélange au mélange réactionnel
• un premier et un deuxième anticorps anti-PF4 et
• un premier constituant et un deuxième constituant d'un système donnant un signal, qui coopère de manière à créer un signal détectable, si le premier constituant et le deuxième constituant du système donnant un signal sont mis à proximité l'un de l'autre dans l'espace,
et dans lequel le premier anticorps anti-PF4 est associé au premier constituant du système donnant un signal ou on l'associe pendant l'incubation du mélange réactionnel, et dans lequel le deuxième anticorps anti-PF4 est associé au deuxième constituant du système donnant un signal ou on l'associe pendant l'incubation du mélange réactionnel.

5. Procédé suivant la revendication 4, dans lequel le premier constituant et le deuxième constituant du système donnant un signal comprennent chacun une phase solide particulaire, et dans lequel on mesure l'agglutination des phases solides particulaires du mélange réactionnel.

6. Procédé suivant la revendication 4, dans lequel le premier constituant du système donnant un signal est un agent chimioluminescent et le deuxième constituant du système donnant un signal est un photosensibilisateur ou inversement et dans lequel on mesure la chimioluminescence du mélange réactionnel.

7. Procédé suivant l'une des revendication s4 à 6, dans lequel on ne soumet pas le mélange réactionnel préparé à un stade de centrifugation pour la séparation des thrombocytes.

8. Procédé de diagnostic d'une thrombocytopénie induite par l'héparine, dans lequel on détecte par un procédé suivant l'une des revendications 1 à 7 la présence d'anticorps de complexe anti-PF4/héparine dans un échantillon de liquide corporel d'un patient.

9. Trousse de test pour effectuer un procédé suivant l'une des revendications 1 à 8, qui contient les constituants suivants :
a. un premier réactif contenant des thrombocytes ;
b. un deuxième réactif contenant un polysaccharide non ramifié fixant PF4 ou un polyanion fixant PF4 ; et
c. un réactif ou plusieurs réactifs de détection de PF4, au moins un réactif contenant un anticorps anti-PF4.

10. Trousse de test suivant la revendication 9, dans laquelle le deuxième réactif
• contient un polysaccharide non ramifié fixant PF4 du groupe héparine, héparine non fractionnée, héparine fractionnée, dextransulfat et fusidane ; ou
• contient un polyanion fixant PF4 du groupe poly(sulfate de vinyle), poly(sulfonate de vinyle), poly(phosphate de vinyle), poly(phosphonate de vinyle), poly(sulfate de styrène) et poly(sulfonate de styrène).
